# EUROPEAN PATENT APPLICATION

(11) **EP 2 106 820 A1**
(43) Date of publication of application: **07.10.2009**
(21) Application number: 08006483.5
(22) Date of filing: 31.03.2008
(51) Int. Cl.: A61M 31/00, A61L 31/14, A61L 29/14

(54) **Expansible biocompatible coats comprising a biologically active substance**

(71) Applicant: Heilmann, Torsten, 07639 Bad Klosterlausnitz (DE)
(72) Inventor: Heilmann, Torsten, 07639 Bad Klosterlausnitz (DE)
(74) Representative: Zwicker, Jörk

(57) **Abstract**

The present invention relates to an expansible hollow part, having at least one opening, which consists of an elastic biocompatible material and which comprises at least one biologically active substance and, optionally at least one matrix compound. The invention also provides a method of producing said expansible hollow part, a medical device covered at least partially with said hollow part, a kit-of-parts comprising said hollow part of the invention and the use of said hollow part as a therapeutic device and for protecting a medical device.

## Description

The present invention relates to an expansible hollow part, having at least one opening, which consists of an elastic biocompatible material and which comprises at least one biologically active substance and, optionally at least one matrix compound. The invention also provides a method of producing said expansible hollow part, a medical device covered at least partially with said hollow part, a kit-of-parts comprising said hollow part of the invention and the use of said hollow part as a therapeutic device and for protecting a medical device.

### BACKGROUND OF THE INVENTION

At present, diseased vessels, vascular constrictions and certain organ failures, e.g. due to a stricture can be treated by systemic administration of pharmaceuticals and by expanding and supporting an affected vessel segment or organ lumen by introducing and expanding a balloon catheter or metal stent either of which may be coated with a suitable pharmaceutical substance or by rinsing the diseased tissue with medicaments, for example, by using a perfusion catheter.

However, above-mentioned systemic administration of pharmaceutical substances generally is achieved by oral administration or by an intra-arterial or intravenous injection. As a consequence, the pharmaceutical substances are released into the blood stream and are distributed not solely to the affected vessel or organ. Thus, merely small amounts of the therapeutic substances will arrive at the vessel segment or organ part which has to be treated. Especially for serious diseases, necessitating a high dose of medicaments to be administered to the diseased vessel or organ, systemic administration may cause harmful side effects since also surrounding healthy vessels and organs are contacted with the pharmaceutical substances. Thus, the effectiveness and dose of medicaments that can be administered is limited.

As mentioned, also balloon catheters and stents can be used to treat a stenosis. However, a mechanical expansion of restricted vessels and organs generally results in relapses of up to 60% of the treated area which will, thus, need a repeated treatment. The reasons for these relapses can be a recoil of the vessel or organ wall directly following treatment or inflammatory processes. A relapse may also be caused by a hyperproliferation of the neointima tissue induced by micro-lacerations of the tissue which occur at the site of mechanical expansion.

To counteract the mentioned recoil of the vessel or organ, metal stents may be implanted which dilate the vessel and organ lumens permanently. However, such stent implantation generally results, similarly to the treatment by balloon expansion, in micro-lacerations of the inner tissue layers which lead to the generation of excess scar tissue which is caused by the hyperproliferation of the neointima as mentioned above. As a consequence, a restenosis, i.e. a recurrent narrowing of the vessels and organs is observed for about 30% of the vessels and organs treated with a stent implantation.

To counteract the mentioned neointima hyperproliferation, anti-proliferative medicaments may be applied. Application of such cytostatic medicaments was attempted by directly coating balloon catheters and metal stents with suitable anti-proliferative pharmaceutical substances or by rinsing of the vessel and organ walls with such anti-proliferative substances.

In case of balloon catheters, the medicaments are generally directly applied to the surface of the balloon. This, however, frequently leads to a premature release of the medicaments from the balloon surface into the bloodstream before they can be brought into contact with the target vessel or organ since said medicaments generally exhibit a poor affinity to the surface of the balloon catheter. Especially hydrophilic substances are prone to be released prematurely from the balloon surface during positioning of the balloon at the area to be treated.

Furthermore, the coating of balloon catheters with pharmaceutical substances frequently leads to local adhesions or encrustations at contact sites between balloon folds which prevents a homogeneous coating of the balloon catheter. Furthermore, due to the tight folding, a balloon catheter has a tendency to self-inflate which causes the medical substance to be stripped off when its surface contacts e.g. the transport protector of the balloon catheter or when it contacts a vessel wall prior to reaching the target site of application in the patient. As a consequence, in most cases, a significant and unpredictable amount of pharmaceutical substance is lost from the balloon catheter before the site of application is reached and, thus, only a reduced and undefined amount of the medicament will be brought into contact with the diseased vessel or organ tissue.

In case pharmaceutical substance coated metal stents are applied, the total surface of the struts of the stent will contact only about 15% of the vessel or organ wall when the stent is brought into its expanded state. Thus, only an insufficient area of affected tissue will be contacted and can be treated with the medicament. Furthermore, metal stents coated with pharmaceutical substances will generally reside permanently in the patient which, in the long run, tends to cause complications such as hypersensitivity, inflammation and thromboses.

Attempts were also made to rinse the diseased vessel region or the diseased organ lumen using a perfusion catheter or a catheter having a double lumen. When using such catheters, however, the pharmaceutical substance is brought into contact with the affected tissue regions via diffusion. Therefore, a large amount of the medicament will not reach the diseased tissue but will be flushed and transported into downstream vessels and organs which leads to a significant exposure of these tissues to cytostatic substances, leading to harmful side effects.

Thus, there is a need for a medical device which can be manufactured with relatively small effort and costs and which allows a homogenous and reproducible localized application of any pharmaceutical substance to a target tissue without the risk that during manufacturing, sterilization, storage, transport or use the pharmaceutical substance either sticks to contact sites between balloon folds which may lead to jamming or a non-homogenous coating and without the risk of any premature release or stripping off of the medical substances from the medical device, e.g. a stent or balloon catheter as mentioned above.

### SUMMARY OF THE INVENTION

To solve above-mentioned problems and further problems associated with the prior art medical devices and methods, the present invention provides an expansible hollow part, having at least one opening, which consists of an elastic biocompatible material and which comprises at least one biologically active substance and, optionally, at least one matrix compound, wherein the expansible hollow part is porous and/or comprises micro-cavities in its surface.

Furthermore, the present invention provides an expansible hollow part, having at least one opening, which consists of an elastic biocompatible material and which comprises at least one biologically active substance, and, optionally, at least one matrix compound, wherein said expansible hollow part is characterized in that it has an inner diameter smaller than 1 cm in its non-expanded state.

Also provided is a an expansible hollow part, having at least one opening, which consists of an elastic biocompatible material and which comprises at least one biologically active substance, and, optionally, at least one matrix compound, wherein said expansible hollow part has a wall strength of smaller than 1 mm in its non-expanded state.

The invention also provides a method of producing an expansible hollow part having at least one opening, which consists of an elastic biocompatible material and which comprises at least one biologically active substance and, optionally, at least one matrix compound, comprising the steps:
(a) expanding the expansible hollow part to at least 110% of its non expanded circumference, and,
(b) contacting the outer surface of the expansible hollow part with at least one biologically active substance and/or at least one matrix compound.

Also comprised is an expansible hollow part producible by the method of the invention.

Also provided is a medical device covered at least partially by the expansible hollow part according to the invention.

Further provided is a kit-of-parts comprising at least one expansible hollow part according to the invention.

The invention provides further a use of an expansible hollow part according to the invention for the preparation of an enhanced balloon catheter for the treatment of a disease or a medical insufficiency selected from the group consisting of a stenosis, a restenosis, a stricture, a defective bypass craft, a thrombosis, a dissection, a tumor, a calcification, an arteriosclerosis, an inflammation, an autoimmune response, a necrosis, an injured anastomosis, a lesion, an allergy, a wart, a hyperproliferation, an infection, a scald, an edema, a coagulation, a cicatrization, a burn, a frostbite, and a lymphangitis.

In a further aspect the invention provides an expansible hollow part according to the invention for the use as a therapeutical device for the treatment of a disease or a medical insufficiency selected from the group consisting of stenosis, restenosis, a stricture, a defective bypass craft, a thrombosis, a dissection, a tumor, a calcification, an arteriosclerosis, an inflammation, an autoimmune response, a necrosis, an injured anastomosis, a lesion, an allergy, a wart, a hyperproliferation, an infection, a scald, an edema, a coagulation, a cicatrization, a burn, a frostbite, and a lymphangitis.

In a further aspect the invention provides a use of an expansible hollow part according to the invention for protecting a medical device or a biological tissue from mechanical stress, thermal stress, chemical stress and/or micro organisms.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.
Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland).
Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.
Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

The object of the present invention is to provide a means for the local delivery of drug/drug combinations from a medical device such as a stent, or a balloon catheter having the advantage that the drug/drug combinations can be applied without any loss of expensive medicaments and that the full surface of the medical device can be utilized, i.e. the medicament can be transferred are-wide from the medical device to the target tissue, thus providing an effective means to counteract e.g. neointimal hyperplasia, restenosis, inflammation and thrombosis.

It is one unexpected finding of the present invention, that an expansible hollow part according to the invention can efficiently be coated with a biologically active substance in such way that said expansible hollow part solves the aforementioned problems.

Thus, in a first aspect, the invention provides an expansible hollow part, having at least one opening, which consists of an elastic biocompatible material and which comprises at least one biologically active substance and, optionally, at least one matrix compound, wherein the expansible hollow part is porous and/or comprises micro-cavities in its surface.

An expansible hollow part of the invention can preferably have the shape of a tubular structure having either one or, more preferably, two open ends. Furthermore, it is preferred that the expansible hollow part of the invention is sterile and/or bioresorbable. Materials which are bioresorbable are known in the art.

In a preferred embodiment, the expansible hollow part of the invention is not a foil. In a further preferred embodiment, the expansible hollow part of the invention does not comprise gelatine.

In a further aspect, the invention relates to an expansible hollow part, having at least one opening, which consists of an elastic biocompatible material and which comprises at least one biologically active substance, and, optionally, at least one matrix compound, wherein said expansible hollow part is characterized in that it has an inner diameter smaller than 3, 2.5, 2.0, 1.5, 1, 0.9, 0.8, 0.7, 0.6, 0.5, 0.4, 0.3, 0.2 or smaller than 0.1 cm in its non-expanded state. Most preferably, the inner diameter is smaller than 1 cm in its non-expanded state when measured at its narrowest section.

Also provided is an expansible hollow part, having at least one opening, which consists of an elastic biocompatible material and which comprises at least one biologically active substance, and, optionally, at least one matrix compound, wherein said expansible hollow part has a wall strength of smaller than 2 mm, 1 mm, 0.9 mm, 0.8 mm, 0.7 mm, 0.6 mm, 0.5 mm, 0.4 mm, 0.3 mm, 0.2 mm or smaller than 0.1 mm in its non-expanded state. Most preferably, the wall strength is smaller than 1 mm.

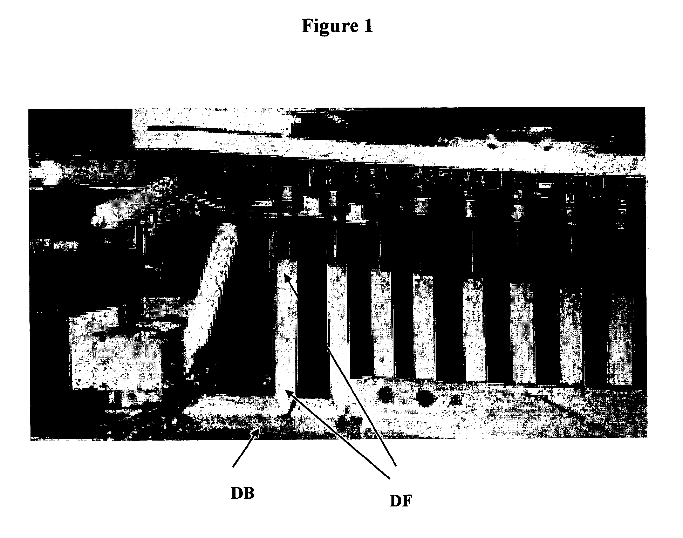

In one example, the expansible hollow part of the invention can be produced, as will be outlined below in greater detail, by dipping a dipping former into a dipping bath (see examples and fig. 1). Preferably, the expansible hollow part of the invention is dried in between the dipping rounds through the use of fans, heaters, blowers or the like or by freeze drying or vacuum drying techniques or the like. Once the expansible hollow part is "dry", the thickness of its wall may be determined utilizing any number of measuring techniques. Preferably, no force is exerted onto the expansible hollow part when measuring the wall strength, i.e. the wall thickness. If a greater wall strength is desired, the expansible hollow part may repeatedly be dipped and dried until the desired thickness is achieved. Preferably, the solvent part of the dipping solution or dipping emulsion is such, that upon successive dippings, the solvent part will not re-dissolve the dried material on the surfaces of the expansible hollow part. It is preferred that non-organic solvents are utilized for the preparation of the dipping solution or dipping suspension. Preferably, the material of the expansible hollow part is selected such that the average tickness of the wall of the material in its relaxed state is at least 1.1, 1.2, 1.3, 1.4, 1.5, 1.6, 1.7, 1.8, 1.9, 2.0, 2.1, 2.2, 2.3, 2.4, 2.5, 3.0, 3.5, 4.0, 4.5, 5.0 times larger than the wall thickness, i.e. wall strength of the material when the expansible hollow part is in the maximally expanded state (i.e. without incurring irreverible structural damage to the material). Preferably, the material of the expansible hollow part of the invention is an amorphous polymer.

As used herein "expansible" refers to the ability of the material of the expansible hollow part according to the invention to reversibly expand its surface when exposed to mechanical stress, i.e. a force causing deformation. Thus, the surface will return to its original, i.e. "relaxed" configuration, when the stress is removed. As used herein "relaxed" means in the absence of any external forces except the average atmospheric pressure present on earth at an altitude of between zero and 500m above sea level. If in a preferred embodyment the expansible hollow part of the invention is a tubular structure, it is preferred that "expansible" means that the tubular structure can be reversibly expanded in its circumference. In a preferred embodyment, the expansible hollow part of the invention is expansible to at least 110%, 115%, 120%, 140%, 160%, 180%, 200%, 400%, 600%, 800%, 1000%, 1200%, 1400%, 1600%, 1800% or to at least 2000% of the circumference of its non-expanded state. Preferably the expansible hollow part according to the invention has an inner diameter smaller than 3, 2.5, 2.0, 1.5, 1, 0.9, 0.8, 0.7, 0.6, 0.5, 0.4, 0.3, 0.2 or smaller than 0.1 cm in its non-expanded state. Most preferably, the inner diameter is smaller than 1 cm in its non-expanded state when measured at its narrowest section.

It is further preferred that the expansible hollow part according to the invention is porous and/or comprises micro-cavities in its surface. As used herein, "porous" or "porosity" refers to the property of a material. If a material is "porous", it comprises pores, i.e. volume of void space interspersed in the material. Preferably, the material of the hollow part according to the invention has a macro porosity, meso and/or micro porosity, wherein macro porosity refers to the presence of pores greater than or equal to 800 nm, 700 nm, 600 nm, 500 nm, 400 nm, 300 nm, 200 nm, 100 nm or greater than or equal to 50 nm in diameter, meso porosity refers to pores greater or equal than 2 nm and less than 50 nm in diameter and micro porosity refers to pores smaller than 2 nm in diameter. Most preferably, the expansible hollow part of the invention has a maximum pore size of 1 µm. Porosity can be measured without undue burden by measuring the pore diameters in a representative cross section of the material using microscopy, such as electron microscopy. Preferably, the porosity of the expansible hollow part according to the invention is such that it is permeable to ethanol. It is noted that the porosity as it is used herein referes to the porositiy of the expansible hollow part in its non-expanded, i.e. relaxed state. While it is considered that the entire material used to produce the expanded hollow part is porous, it is also possible that only a surface region shows the indicated macro, meso or micro porosity, preferably the outer surface.

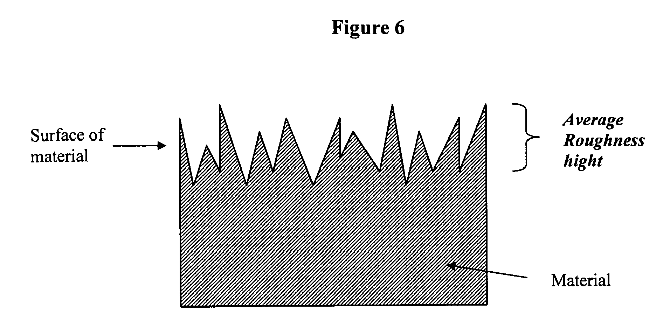

As mentioned, the material of the expansible hollow part according to the invention preferably comprises micro-cavities in its surface. This means that the material surface is rough, having an average roughness height of at least 2 nm, 10 nm, 100 nm, 1 µm, 10 µm, 20 µm, 30 µm, 40 µm, 50 µm, 60 µm, 70 µm, 80 µm, 90 µm, or of at least 100 µm. Roughness height is the average altitude of surface irregularities of the material (see fig. 6). The average roughness height of said material is preferably at least 1.1, 1.2, 1.3, 1.4, 1.5, 1.6, 1.7, 1.8, 1.9, 2.0, 2.1, 2.2, 2.3, 2.4, 2.5, 3.0, 3.5, 4.0, 4.5, 5.0 times smaller in the expanded state than in the relaxed state of the expansible hollow part according to the invention. The micro-cavities in the surface of the expansible hollow part can be generated by e.g. blasting the surface with sand, glass beads, or water. The pore size can be adjusted by, e.g. chosing a suitable material composition of the expansible hollow part of the invention. Detailed instructions can be found, for example in an article by Steve Jons et. al., "Porous latex composite membranes: fabrication and properties" published in the Journal of Membrane Science, volume 155, issue 1, 31 March 1999, p. 79-99. To generate pores and/or micro-cavities, the material of the expansible hollow part of the invention, e.g. a latex composition, may also be mechanically foamed before casting, mechanically punctured, or temporary fillers may be placed in the material before it is cast which are dissolved or digested out subsequently.

The use of materials which have the above-described prefered porosity and/or microcavities, for the production of an expansible hollow part according to the invention leads to the surprising effect that when an expansible hollow part of the invention is coated in its expanded state with a biologically active substance, the biologically active substance will effectively enter the pores and/or microcavities of the expansible hollow part.

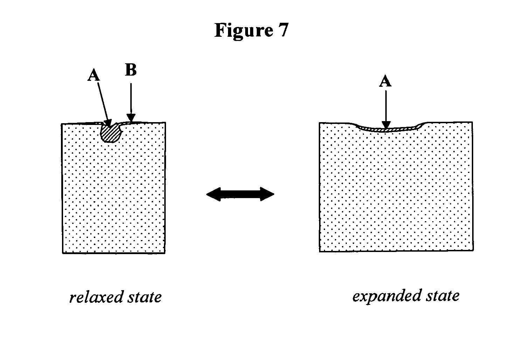

When an expansible hollow part according to the invention is relaxed after the coating process, it has been found that, unexpectedly, a significant amount of the biologically active substance will reside in the pores and/or microcavities of the hollow part (see also figure 7). The pores and/or microcavities protect the biologically active substance from being sloughed off or from being prematurely dissolved away from the expansible hollow part of the invention. Thus, for example, a balloon catether covered at least partially by the expansible hollow part of the invention can be used for the local administration of drugs, agents or compounds. As will be explained below, also stents can be used in conjunction with an expansible hollow part of the invention to obtain improved therapeutic effects. Also higher tissue concentrations of the drugs, agents or compounds are achievable since hardly any biologically active substance is lost prematurely due to mechanical stress or solvent exposure. The biologically active substance is effectively released when the expansible hollow part of the invention is expanded at the target site and a diseased vessel or organ region is brought into contact with the expanded surface of the expansible hollow part of the invention. As only a very small amount of the biologically active substance is released into the blood stream, systemic toxicity is reduced and the therapeutic ratio (efficacy/toxicity) of the biologically active substance - preferably an antirestenosis, anti-inflammatory or anti-thrombotic agent - is improved. Furthermore, due to the increased concentration of the biologically active substance that is achievable in the affected tissue, a single treatment may suffice with better patient compliance.

Accordingly, preferred is an expansible hollow part of the invention, wherein more than 10%, 20%, 30%, 40%, 50%, 60%, 70%, 80% or more than 90% of the biologically active substance is located in the pores and/or in the micro-cavities of the outer surface of the expansible hollow part, preferably, when the expansible hollow part of the invention is at its relaxed state. The respective amount located in the pores and/or micro-cavities can be increased by increasing the expansion of the expansible hollow part during application of the biologically active substance and/or increase the depth of the micro-cavities and/or number and size of the pores. One technical effect of this preferred embodiment is that the biologically active substance is better protected against mechanical and chemical stress when the expansible hollow part according to the invention is in its relaxed state.

A skilled person can, without undue burden determine how much of the biologically active substance is located in the pores and/or in the micro-cavities. In one example, microscopy, preferably electron microscopy is used to determine the average amount of biologically active substance which is located in the pores and/or in the micro-cavities versus the amount which adheres to the remaining surface (see fig. 7). For this microscopy approach, a representative cross section through an expansible hollow part of the invention is used. Alternatively, the amount of biologically active material which is located in the pores and/or micro-cavities can be determined by measuring the dissolution of the biologically active substance. Thereby the skilled person compares what amount of the biologically active substance is dissolvable from an expanded hollow part versus a non-expanded hollow part in a fixed period of time. For example, if in one example, the amount of the biologically active substance which can be dissolved in a given period of time under identical experimental conditions (same temperature, same solvent, same area of surface exposed to solvent) from an expansible hollow part according to the invention in its relaxed state is 80% less than the amount that can be dissolved when the expansible hollow part is in its expanded state, then it is defined that 80% of the biologically active substance is located in the pores and/or in the micro-cavities. As used herein, "expanded state" refers to a state, wherein the expansible hollow part of the invention is expanded to at least 110%, 115%, 120%, 140%, 160%, 180%, 200%, 400%, 600%, 800%, 1000%, 1200%, 1400%, 1600%, 1800% or to at least 2000% of its circumference of its non-expanded state. As used herein "circumference" refers to the largest possible circumference of the expansible hollow part. Most preferably, the circumference of the largest possible imaginary sphere is used which fits inside, i.e. into the lumen of the expansible hollow part of the invention. When determining what percentage of biologically active substance is located in said pores and/or in the micro-cavities it is preferred that two comparable copies of an expansible hollow part of the invention are submerged in a suitable solvent in a solvent bath for preferably 1, 2, 3, 4, 5, 6, 7, 8, 9, or 10 minutes. The solvent may be selected from the group consisting of distilled water, phosphate buffered saline, whole blood, blood serum and an organic solvent such as an alcohol, preferably ethanol. The most preferred assay conditions that can be used when determining the amount of biologically active substance that is located in the pores and/or in the micro-cavities comprises a time period of 5 minutes, room temperature and the comparison of a hollow part in its relaxed state with a hollow part which is expanded to between 400% and 600% and most preferably to about 500% of the circumference of its non-expanded (i.e. relaxed) state.

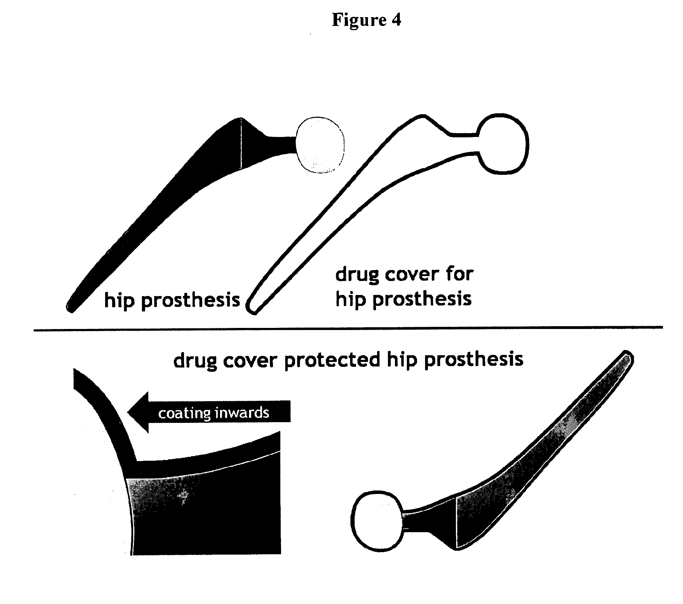

A further preferred embodiment of the invention is the expansible hollow part of the invention, wherein more than 10%, 20%, 30%, 40%, 50%, 60%, 70%, 80% or more than 90% of the biologically active substance is located in the pores and/or in the micro-cavities of the inner surface of the expansible hollow part. Such preferred embodiment is obtainable, as will also be described below, by everting the expansible hollow part after it has been coated with the biologically active substance. The skilled person can determine the amount of biologically active substance which is located in the pores and/or in the micro-cavities as described above. Expansible hollow parts which are coated on the inside are particularly useful for wrapping medical devices or a biological sample for storage and/or transport (see also description below and figure 4).

Another preferred embodiment of the invention is the expansible hollow part of the invention, wherein more than 10%, 20%, 30%, 40%, 50%, 60%, 70%, 80% or more than 90% of the biologically active substance is located in the pores and/or in the micro-cavities of the inner and outer surface of the expansible hollow part.

Also preferred is the expansible hollow part of the invention, wherein more than 10%, 20%, 30%, 40%, 50%, 60%, 70%, 80% or more than 90% of a first biologically active substance is located in the pores and/or in the micro-cavities of the inner surface of the expansible hollow part and wherein more than 50% and preferably more than the same lower boundary value as defined for the first biological substance above, of a second biologically active substance is located in the pores and/or in the micro-cavities of the outer surface of the expansible hollow part.

When the expansible hollow part of the invention comprises pores and/or micro-cavities then it is preferred that the depth of the micro-cavities and/or number and size of the pores is such that the expansible hollow part of the invention comprises at least 0.5, 0.6, 0.7, 0.8, 0.9, 1, 2, 3, 4, 5, or at least 6 µg of the respective biologically active substance per square millimetre surface in its non-expanded state.

As described above, the expansible hollow part of the invention preferably comprises or consists of an amorphous polymer. In a more preferred embodiment, the elastic biocompatible material of the expansible hollow part according to the invention consists, comprises or essentially consists of a material selected from the group consisting of:
latex, polyvalerolactone, poly-ε-decalactone, polylactic acid, polyglycol acid, polylactide, polyglycolide, co-polymer of polylactide and polyglycolide, poly-ε-caprolactone, polyhydroxy butyric acid, polyhydroxybutyrate, polyhydroxyvalerate, polyhydroxybutyrate-co-valerate, poly(1,4-dioxan-2,3-dione), poly(1,3-dioxan-2-one), poly-para-dioxanone, polyanhydride, polymaleicacidanhydride, polyhydroxymethacrylate, fibrin, polycyanoacrylate, polycaprolactondimethylacrylate, poly-β-maleic acid, polycaprolactonbutylacrylate, multiblockpolymers made of oligocaprolactondiole and oligodioxanondiole, polyetherestermultiblockpolymers made from PEG and polybutylenterephtalate, polypivotolactone, poly-glycolic acid trimethylcarbonate polycaprolactonglycolide, poly(g-ethylglutamate), poly(dth-iminocarbonate), poly(dte-co-dt-carbonat), poly( bisphenol A-iminocarbonate), polyorthoester, poly- glycolic acid -trimethylcarbonate, polytrimethylcarbonate polyiminocarbonate, poly(n-vinyl)-pyrrolidone, polyvinylalcohols, polyesteramide, glycolized polyester, polyphosphoester, polyphosphazene, poly(p-carboxyphenoxy)propane], polyhydroxypentanoic acid, polyethylenoxidpropylenoxid, polyurethane, polyurethane comprising amino acids, polyetherester like polyethyleneoxide, polyalkeneoxalate, lipids, carrageenane, fibrinogen, starch, collagene, protein-based polymers, polyaminoacids, zein, polyhydroxyalkanoate, pectic acid, actinic acid, carboxymethylsulfate, albumine, hyaluronic acid, chitosane, heparanesulfate, heparine, chondroitinsulfate, dextrane, β-cyclodextrine, copolymers comprising PEG and polypropyleneglycole, gummi arabicum, guar, gelatine, collagen-n-hydroxysuccinimide, phospholipids, polyacrylic acid, polyacrylate, polymethylmethacrylate, polybutylmethacrylate, polyacrylamide, polyacrylonitrile, polyamide, polyetheramide, polyethyleneamine, polyimide, polycarbonate, polycarbourethane, polyvinylketone, polyvinylhalogenide, polyvinylidenhalogenide, polyvinylether, polyisobutylene, aromatic compounds comprising a polyvinyl functional group, polyvinylester, polyvinylpyrollidone, polyoxymethylene, polytetramethyleneoxide, polyethylen, polypropylen, polytetrafluorethylen, polyetherurethane, silicon- polyetherurethane, silicon-polyurethane, silicon-polycarbonat-urethane, polyolefin-elastomers, epdm-rubber, fluorosilicone, carboxymethylchitosane, polyaryletheretherketone, polyetheretherketone, polyethylenterephtalate, polyvalerate, carboxymethylcellulose, cellulose, rayon, rayontriacetate, cellulosenitrate, celluloseacetate, hydroxyethylcellulose, cellulosebutyrate, celluloseacetatebutyrate, ethylvinylacetate, polysulfone, epoxy-resin, abs-resin, silicone like polysiloxane, polydimethylsiloxane, polyvinylhalogens, cellulose-ether, cellulose-triacetate, copolymers mixtures and derivatives thereof. As used herein, the term "derivative" refers to a chemical compound or molecule made from a parent compound or molecule by one or more chemical reactions. Preferably, however, the parent molecule must still be comprised in its structure in a "derivative". Additionally, the derivative preferably has a molecular weight which is not greater than twice the molecular weight of the parent compound or parent monomer if the compound is comprised in a polymer.

In a more preferred embodiment, the elastic biocompatible material of the expansible hollow part according to the invention is selected from the group consisting of natural rubber, synthetic polyisoprene, a copolymer of isobutylene and isoprene, a halogenated butyl rubbers , a polybutadiene, a styrene-butadiene rubber, a copolymer of polybutadiene and acrylonitrile, a hydrogenated nitrile rubber, a chloroprene rubber, a polychloroprene, latex, a neoprene, a baypren. In another preferred embodyment the elastic biocompatible material is selected from a saturated rubber that cannot be cured by sulfur vulcanization such as ethylene propylene rubber, ethylene propylene diene rubber, epichlorohydrin rubber, polyacrylic rubber, silicone rubber, fluorosilicone rubber, fluoroelastomers, perfluoroelastomers, polyether block amides, chlorosulfonated polyethylene, ethylene-vinyl acetate, a resilin proteins and an elastin protein. Also either carbon black or silica may be added to the elastic biocompatible material of the expansible hollow part according to the invention up to a concentration of about 30 percent by volume which will raise the elastic modulus of the rubber material by a factor of about two to three.

In a most preferred embodiment, the elastic biocompatible material of the expansible hollow part according to the invention is latex, especially Guayule (Parthenium argentatum) latex which is hypoallergenic.

A further preferred embodiment of the invention is the expansible hollow part according to the invention, wherein the biologically active substance is selected from the group consisting of: a vasoconstrictor, a vasodilatator, a muscle relaxant, an antimycotic, a cytotoxic agent, a prodrug of a cytotoxic agent, a virustatic, a physiological or pharmacological inhibitor of mitogens, a cytostatic, a chemotherapeutic, an adrenocorticostatic, a β-adrenolytic, an androgen or antiandrogen, an antianemic, an anabolic, an anaesthetic, an analeptic, an antiallergic, an antiarrhythmic, an antiarterosclerotic, an antibiotic, an antifibrinolytic, an anticonvulsive, an angiogenesis inhibitor, an anticholinergic, an enzyme, a coenzyme, an antihistaminic, an antihypertensive, an antihypotensive, an anticoagulant, an antiseptic, an antihemorrhagic, a beta-receptor antagonist, a calcium channel antagonist, an antimyasthenic, an antiphlogistic, an antipyretic, a glucocorticoid, a haemostatic, an immunoglobuline or its fragment, a chemokine, a mitogen, a cell differentiation factor, a hormone, an immunosuppressant, an immunostimulant, a mineralcorticoid, a narcotic, a vector, a peptide, a (para)-sympathicomimetic, a (para)-sympatholytic, a protein, a cell, a sedating agent, a spasmolytic, a wound-healing substance, and combinations thereof.

In a more preferred embodiment, the biologically active substance of the expansible hollow part is selected from the group consisting of a RNA-oligonucleotide, a DNA-oligonucleotide, β-estradiol, 1,11-dimethoxycanthin- 6-on, 12-beta-hydroxypregnadien 3,20-dion, 13,18-dehydro-6-alpha- senecioyloxychaparrin, 14-dehydroagrostistachin, 17-hydroxyagrostistachin, 1-hydroxy-11-methoxycanthin-6-on, 2-chloro-deoxyadenosin, 3-deazaadenosin, 4,7-oxycycloanisomelic acid, 4-hydroxyoxycyclophosphamid, abciximab, aceinhibitors like captopril, acemetacin, acetylvismion B, aclarubicin, actinomycin, ademetionin, adriamycin, aescin, afromoson, agroskerin, agrostistachin, akagerin, aldesleukin, amidoron, aminoglutethemid, amsacrin, anakinra, anastrozol, anemonin, angiopeptin, anopterin, antimykotika, antiprozoale agentien like chloroquine, antisense oligonucleotide, antithrombin, antithrombotika, apocymarin, apoptosis inhibitors, apoptosis modulators like p65, argatroban, aristolactam-aii, Aristolochia Acid, ascomycin, asparaginase, aspirin, atorvastatin, auranofin, azathioprin, azelastin, azithromycin, baccatin, baccharinoide B1, B2, B3 and B7, bacitracin, bafilomycin, barringtogenol-C21-angelat, basiliximab, batimastat, bendamustin, benzocain, berberin, betamethason, betulin, betulinic acid, bevacizumat, bfgf-antagonisten, bilobol, biorest, bisparthenolidin, bivalirudin, bleomycin, b-myc-antisense, bombrestatin, bosentan, boswellic acid, bruceanole A, B and C, bruceantinoside C, bryophyllin A, busulfan, c myc specific antisense oligonucleotide, cadherine, camptothecin, capecitabin, carboplatin, carmustin, cefaclor, cefazolin, cefotixin tobramycin, celecoxib, cepharantin, cerivastatin, CETP-inhibitoren, cheliburinchlorid, chlorambucil, chloroquinphosphat, cictoxin, ciglitazone, cilazapril, cilostazol, ciprofloxacin, cisplatin, cladribin, clarithromycin, clotrimazol, colcemid, colchicin, concanamycin, coronarin A, B, C and D, coumadin, cox-2-lnhibitor, C-proteinase inhibitors, c-type natriuretic peptide (cnp), cudraisoflavon a, curcumin, cyclophosphamid, cyclosporin A, cymarin, cytarabin, cytochalasin A-E, cytokinine inhibitors, D-24851 (calbiochem), dacarbazin, daclizumab, dactinomycin, daphnoretin, dapson, daunorubicin, deoxypsorospermin, desacetylvismion A, desulfurated and n-reacetylated heparin (hemoparin®), dexamethason, diclofenac, dicloxacyllin, anti proliferative compounds, anti-cancer compounds, dihydronitidin, dihydrousambaraensin, disopyrimid, disulferam, docetaxel, doxorubicin, dunaimycin, effusantin A, eicosanoide, ellipticin, enalapril, endothelinantagonistenenoxoparin, epicatechingallat, epigallocatechingallat, epirubicin, epothilone A and B, erythromycin, estradiol, estradiolbenzoate, estradiolcypionate, estramustin, estriol, estron, etanercept, ethinylestradiol, etobosid, etoposid, everolimus, excisanin A and B, exemestan, faktor Xa-inhibitor antibody, filgrastim, flecainid, flucytosin, fludarabin, fludarabin-5'-dihydrogenphosphate, fluorouracil, fluroblastin, fluvastatin, folimycin, formestan, fosfestrol, a free nucleic acid, ganciclovir and zidovudin, gemcitabin, gentamycin, tissue-plasminogen-activator, ghalakinosid, ginkgol, ginkgolic acid, glykosid 1a, gpllb/llla-platelet membrane receptor, griseofulvin, guanidylcyclasestimulator, inhibitor of metalloproteinase-1 and 2, halofuginon, helenalin, heparin, hirudin, histaminantagonisten, hydrocortison, hydroxyanopterin, hydroxycarbamid, hydroxychloroquin, hydroxyusambarin, ibuprofen, idarubicin, ifosfamid, igf-1, indanocine, indicin, indicin-n-oxid, indomethacin, inotodiol, interferon A, irinotecan, isobutyrylmallotochromanol, isodeoxyelephantopin, iso-lridogermanal. maytenfoliol, josamycin, justicidin A and B, a terpenoide like kamebakaurin and hippocaesculin, kamebaunin, ketoconazol, ketoprofen, carbonsuboxides (mcs) and macrocyclic oligomers thereof, lanograstim (r-hug-csf), lapachol, L-arginine, lariciresinol, larreatin, lasiocarpin, leflunomid, letrozol, leukamenin a and b, levomenthol, lidocain, liriodenin, liriodenin, lisinopril, lomustin, lonazolac, longikaurin b, losartan, lovastatin, lycoridicin, macrogol, malloterin, mallotochromanol, mansonin, maquirosid A, marchantin A, margetin, maytansin, medroxyprogesteron, mefenamin acid, mefloquin, melatonin, meloxicam, melphalan, mercaptopurin, methotrexat, methoxylariciresinol, methylsorbifolin, metronidazol, miconazol, midecamycin, miltefosin, mitomycin, mitoxanthrone, mitoxantron, mizoribine, mofebutazon, molgramostim (rhuGM-csf), molsidomin, monoclonal antibodies, m-prednisolon, mutamycin, mycophenolatmofetil, mycophenolic acid, myrtecain, naproxen, natriumaurothiomalat, steroids like inotodiol, NFkB, NF-kB or Bcl-xL-antisense-oligonukleotides, non-steroid compounds (nsaids) like fenoporten, nifedipin, nimustin, nitidinchlorid, nitroprusside, nocadazole, NO-donors like pentaerythrityltetranitrate and syndnoeimine, nonivamid, virus particles comprising oligonucleotides, nystatin, o-carbamoylphenoxyaceticacid, ovatodiolide, oxaceprol, oxacillin, oxaliplatin, oxoushinsunin, paclitaxel, 6-a-hydroxy-paclitaxel, pancratistatin, pcna ribozyme, pdgf-antagonists like triazolopyrimidin and seramin, pegasparase, peginterferon a-2b, penicillamin, penicilline like dicloxacillin, pentostatin, periplocosid a, antiviral compounds like phenylbutazon and acyclovir, pioglitazone, piroxicam, pitavastatin, plaminogen-activator inhibitor- 1, plasminogen-activator inhibitor-2, podophyllotoxin, podophyllicacid-2-ethylhydrazid, polidocanol, PPACK, pravastatin, probucol, procainimid, procarbazin, prolylhydroxylase inhibitors, propafenon, prostacyclin, prostaglandine, protamin, protoanemonin, prourokinase, psycorubin, quinidin, quinin, rapamycin, regenilol, restenase, retinoic acid, r-hirudin, ricin a, rosiglitazone, rosuvastatin, roxithromycin, s 100 protein, sanguinarin, scopolectin, sculponeatin C, selectin (cytokinantagonist), serotoninblocker, shikonin, simvastatin, sinococulin A and B, sirolimus (rapamycin), smc-proliferation-lnhibitor-2w, s-nitrosoderivate, somatostatin, sotolol, sphatheliachromen, spiramycin, β-estradiol, β-lapachon, β-sitosterin, statine, staurosporin, stizophyllin, streblosid, streptokinase, strychnopentamin, strychnophyllin, sulfasalazin, sulfonamide, syringaresinol, tacrolimus, tamoxifen, taxamairin a and b, taxotere, teepolyphenole, teniposid, terbinafin, tetracyclin, tezosentan, thioguanin, thioproteaseinhibitoren, thiotepa, tocopherol tranilast, tomenphantopin A and B, topotecan, tranilast, transresveratrol, trapidil, tremozolomid, treosulfan, tretinoin, triamcinolon, triptolid, troleandomycin, tropfosfamid, tubeimosid, tyrosin-kinase-inhibitors like tyrphostine, umbelliferon, urokinase, ursol acid, usambarensin, usambarin, valsartan, vapiprost, vasodilators like dipyramidol, VEGF-inhibitors, verapamil, vinblastin, vincristin, vindesin, vinorelbin, vismion A and B, vitronectin-receptor antagonists, warfarin, antibiotika like cefadroxil, yadanzioside N and P, zeorin, mixtures and derivatives thereof.

In a further preferred embodiment, the expansible hollow part of the invention comprises at least 0.5, 0.6, 0.7, 0.8, 0.9, 1, 2, 3, 4, 5, or at least 6 µg of the biologically active substance per square millimetre surface in its non-expanded state, irrespective whether said expansible hollow part comprises comprises pores and/or micro-cavities or not.

As mentioned, the expansible hollow part of the invention may optionally also comprise a matrix compound. This matrix compound is useful to e.g. improve the affinity and/or adhesiveness of the hollow part with respect to a biologically active substance. Thus, preferred is the expansible hollow part according to the invention, wherein the matrix compound is selected from a group consisting of polyvalerolactone, poly-ε-decalactone, polylactic acid, polyglycolic acid, polylactide, polyglycolide, copolymers of polylactide and polyglycolide, poly- ε-caprolactone, polyhydroxylbutyric acid, polyhydroxybutyrate, polyhydroxyvalerate, polyhydroxybutyrate-co-valerate, poly(1,4-dioxane-2,3-dione), poly(1,3-dioxane-2-one), poly-para-dioxanone, polyanhydride, polymaleicacidanhydride, polyhydroxymethacrylate, fibrin, polycyanoacrylate, polycaprolactondimethylacrylate, poly-b-maleic acid polycaprolactonbutylacrylate, multi-block polymers of oligocaprolactondiole and oligodioxanonediole, polyetherestermultiblockpolymers made of peg und polybutylene terephthalate, polypivotolactone, polyglycolic acid trimethylcarbonate polycaprolactonglycolide, poly(g-ethylglutamate), poly(dth-lminocarbonate), poly(dte-co-dt-carbonate), poly( bisphenol a-iminocarbonate), polyorthoester, polyglycolic acid trimethylcarbonate, polytrimethylcarbonate, polyiminocarbonate, poly(n-vinyl)-pyrrolidone, polyvinylalcohol, polyesteramide, glycolated polyester, polyphosphoester, polyphosphazene, poly[p-carboxyphenoxy)propane], polyhydroxypentanoic acid, polyanhydride, polyethyleneoxidepropyleneoxide, polyurethane, polyurethane having amino acids in the backbone, polyetherester like polyethyleneoxide, polyalkeneoxalate, lipid, carrageenane, fibrinogen, starch, collagene, polymers comprising protein, protein, polyaminoacid, synthetic polyaminoacid, zein, polyhydroxyalkaneoate, pectic acid, actinic acid, carboxymethylsulfate, albumin, hyaluronic acid, chitosan und derivatives thereof, heparanesulfate und ist derivatives, heparine, chondroitinsulfate, dextrane, β-cyclodextrine, copolymers of peg and polypropyleneglycol, gummi arabicum, guar, gelatine, collagen-n-hydroxysuccinimide, phospholipid, polyacrylic acid, polyacrylate, polymethyl methacrylate, polybutyl methacrylate, polyacrylamide, polyacrylonitrile, polyamide, polyetheramide, polyethyleneamine, polyimide, polycarbonate, polycarbourethane, polyvinylketone, polyvinylhalogenide, polyvinylidenhalogenide, polyvinylether, polyisobutylene, polyvinyl compounds, polyvinyl ester, polyvinylpyrollidone, polyoxymethylene, polytetramethylene oxid, polyethylene, polypropylene, polytetrafluoroethylene, polyetherurethane, silicone-polyetherurethane, silicone-polyurethane, silicone-polycarbonate-urethane, polyolefin, polyisobutylene, epdm-rubber, fluorosilicone, carboxymethylchitosane, polyaryletheretherketone, polyetheretherketone, polyethylene terephthalat, polyvalerate, carboxymethylcellulose, rayon, rayontriacetate, cellulose nitrate, cellulose acetate, hydroxyethylcellulose, cellulosebutyrate, celluloseacetatbutyrate, ethylvinylacetate, polysulfone, epoxy resin, abs-resin, silicone like polysiloxane, polydimethylsiloxane, polyvinylhalogene, cellulose ether, cellulose triacetate, chiosane, combinations and copolymers of combinations thereof.

In the following, methods are provided which are useful to produce an expansible hollow part according to the invention.

Thus, in one aspect, the invention provides a method of producing an expansible hollow part having at least one opening, which consists of an elastic biocompatible material and which comprises at least one biologically active substance and, optionally, at least one matrix compound, comprising the steps:
(a) expanding the expansible hollow part to at least 101%, 103%, 105%, 106%, 108%, 110%, 115%, 120%, 130%, 140%, 150%, 160%, 170%, 180%, 190%, or to at least 200% of its non expanded circumference, and,
(b) contacting the outer surface of the expansible hollow part with at least one biologically active substance and/or at least one matrix compound.

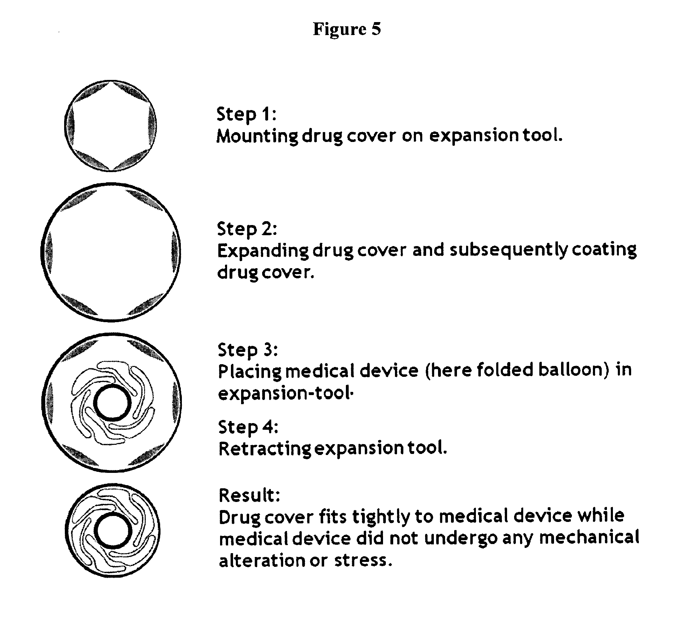

Preferably, the contacting in step (b) is carried out using a method selected from the group consisting of dipping, spraying and printing such as inkjet printing. In one preferred embodiment, step (b) is carried out at room temperature. The expansion in step (a) is preferably carried out using an expansible medical device (such as a stent or a balloon catheter) or a mechanical tool. An example for a suitable mechanical tool is provided in figure 5.
In a preferred embodiment, the method of the invention further comprises at least one additional step selected from the steps consisting of:
(c) relaxing the expansible hollow part so that its circumference is smaller than, preferably 1%, 3%, 5%, 10%, 20%, 30% smaller than the circumference of the expansible hollow part in step (a);
(d) before carrying out step (a), forming the expansible hollow part in a dipping bath comprising the elastic biocompatible material in liquefied form and optionally, a biologically active substance; and
(e) mechanically everting the expansible hollow part.

Methods to form a hollow part using a dipping bath is known in the prior art. One example is shown in figure 1.

It is preferred that step (a) is carried out before step (b), i.e. that the expansible hollow part of the invention is coated with the biologically active substance when it is in its expanded state. Using this procedure, a larger amount of biologically active substance enters micro-cavities and/or pores on the hollow part, leading to several advantages which are described above.

In another preferred embodiment of the method according to the invention steps (a) and (b) or (b) and (c) are carried out simultaneously.
Also preferred is the method according to the invention, wherein
(i) step (e) is carried out before step (a) and/or after step (b) and/or after step (c);
(ii) steps (d), (a) and (b) are carried out in that order;
(iii) steps (d), (e), (a) and (b), are carried out in that order; or
(iv) steps (b), (e), (b) are carried out in that order, whereby in the first step (b) a different biologically active substance is used than the second step (b).

In a further aspect, the invention provides an expansible hollow part producible by the method of the invention.

It is to be understood that the sequence of steps described in the preferred embodiment above includes further preferred embodiments, wherein additional steps selected from the steps (a) thorough (e) are carried out in one or in several instances in between the individual steps of the above indicated sequence of steps. For example, a preferred embodiment of feature (iv) above is a sequence of steps (a), (b), (e), (a), (b) which is carried out in that order.

Also provided is a medical device covered at least partially by the expansible hollow part according to the invention. In a preferred embodiment, the medical device is selected form the group consisting of a stent, a balloon catheter, a probe, a prosthesis, an endoscope, a pace maker, a heart defibrillator and a perfusion catheter. In preferred embodiments the stent is a stent prosthesis. Also preferred is a medical device according to the invention, wherein the balloon catheter is capable of emitting an electromagnetic radiation and/or a vibrational mechanical energy. Also preferred is a medical device according to the invention, wherein said expansible hollow part and said medical device are in contact but do not adhere to each other.

Preferably, a balloon catheter is covered by the expansible hollow part according to the invention. Thus, when the balloon catheter is inflated, the expansible hollow part of the invention is expanded and the biologically active substance contacts the vessel or organ wall. This allows an efficient transfer of the biologically active substance from the hollow part to the tissue. In an alternative preferred embodiment, a stent can be used which is placed between the balloon catheter and the expansible hollow part or, alternatively, over the expansible hollow part which covers the balloon catheter. Thus, preferred is a medical device according to the invention, wherein the medical device is a balloon catheter; and wherein the expansible hollow part is covered at least partially by a stent. Also preferred is a medical device according to the invention, wherein the medical device is a stent; and wherein the stent is on a balloon catheter. When stents are used in combination with an expansible hollow part of the invention they may themselves be coated with a therapeutic biologically active substance or not. In any case, the region of tissue that can be brought into contact with the biologically active substance will be significantly higher (up to 75% more surface) than when a stent is used in the absence of a hollow part. When the transfer of a given biologically active substance into the target tissue is inefficient, the contact time, i.e. the time period in which the surface of the expansible hollow part contacts the tissue, may be increased. However, when using regular balloon catheters, the contact time preferably does not exceed 1 minute when the affected vessel is located in the torso and does not exceed 10 minutes when the target tissue is in a distal body region, such as a leg. Otherwise the patient may suffer an acute heart failure. To further increase the contacting time, perfusion catheters can be used in combination with the expansible hollow part of the invention.

In one preferred embodiment, the balloon catheter of the medical device according to the invention comprises a hot balloon, a cold balloon, an occlusion balloon, a valvuloplasty-balloon and/or a protection device.

Most preferred is a medical device according to the invention, wherein the balloon catheter is a perfusion catheter which comprises a hot balloon or a cold balloon.

The preferred use of a hot balloon in combination with an expansible hollow part of the invention allows the practitioner to further increase the amount of biologically active substance which is transferred to the affected tissue. The hot balloon is preferably heated to a temperature of between 45°C and 70°C for the time period, when the expansible hollow part is in contact with the target tissue. This temporary heat shock will render the contacted tissue more permeable for the at least one biologically active substance which is comprised on the expansible hollow part of the invention. Thus, also the contacting times can be reduced when applying a heat shock, reducing the stress on the patient. Most biologically active substances tolerate a short exposure to a temperature between 45°C and 70°C. A preferred biologically active substance which can be used with a hot balloon catheter is a mitotic inhibitor selected from the group consisting of a taxane such as paclitaxel or docetaxel; a vinca alkaloid such as vinblastine, vincristine, vindesine or vinorelbine; colchicine and derivatives thereof.

In another preferred embodiment, the invention provides a medical device according to the invention, wherein the balloon catheter and the expansible hollow part are connected to each other by at least one clamping piece. A clamping piece is any mechanical device tethering the balloon catheter to the expansible hollow part, for example a thread.

In a further preferred embodiment, the invention provides a medical device according to the invention, wherein the circumference of the medical device in its non-expanded state is greater than the inner circumference of the expansible hollow part in its non-expanded state.

The invention also provides in one aspect a kit-of-parts comprising at least one expansible hollow part according to the invention and at least one medical device, preferably a medical device selected from the group consisting of a stent, a balloon catheter, a probe, a prosthesis, an endoscope, a pace maker, a heart defibrillator and a perfusion catheter. In preferred embodiments, the stent is a stent prosthesis.

Also preferred is the kit-of-parts according to the invention, wherein the balloon catheter comprises a hot balloon, a cold balloon, an occlusion balloon, a valvuloplasty-balloon or a protection device.

Further preferred is the kit-of-parts according to the invention, wherein the balloon catheter is capable of emitting an electromagnetic radiation and/or a vibrational mechanical energy.

In a preferred embodiment of the kit-of-parts according to the invention, the circumference of the medical device in its non-expanded state is greater than the inner circumference of the expansible hollow part in its non-expanded state. This allows a tight-fit of the expansible hollow part onto the medical device.

In a further aspect, the invention provides the use of an expansible hollow part according to the invention for the preparation of an enhanced balloon catheter for the treatment of a disease or a medical insufficiency selected from the group consisting of a stenosis, a restenosis, a stricture, a defective bypass craft, a thrombosis, a dissection, a tumor, a calcification, an arteriosclerosis, an inflammation, an autoimmune response, a necrosis, an injured anastomosis, a lesion, an allergy, a wart, a hyperproliferation, an infection, a scald, an edema, a coagulation, a cicatrization, a burn, a frostbite and a lymphangitis. In a further preferred embodiment of the use according to the invention, the disease or medical insufficiency may be a bone injury.

In another aspect, the invention provides an expansible hollow part according to the invention for the use as a therapeutical device for the treatment of a disease or a medical insufficiency selected from the group consisting of stenosis, restenosis, a stricture, a defective bypass craft, a thrombosis, a dissection, a tumor, a calcification, an arteriosclerosis, an inflammation, an autoimmune response, a necrosis, an injured anastomosis, a lesion, an allergy, a wart, a hyperproliferation, an infection, a scald, an edema, a coagulation, a cicatrization, a burn, a frostbite and a lymphangitis. In a further preferred embodiment, the expansible hollow part according to the invention may also be used as a therapeutical device for the treatment of a bone injury.

In preferred embodiments of the aforementioned medical use or expansible hollow part of the invention, the stenosis is selected from the group consisting of pyloric stenosis, biliary tract stenosis, phimosis, hydrocephalus, stenosing tenosynovitis, spinal stenosis and subglottic stenosis.

The invention also refers to the use of an expansible hollow part according to the invention for protecting a medical device or a biological tissue from mechanical stress, thermal stress, chemical stress and/or micro organisms. Preferred medical devices that can be protected are selected from the group consisting of a stent, a balloon catheter, a probe, a prosthesis, an endoscope, a pace maker, a heart defibrillator and a perfusion catheter. A preferred biological tissue that can be conserved and/or protected using the expansible hollow part of the invention is an organ, wherein the organ is preferably not in a living human being.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.
The following figures and examples are merely illustrative of the present invention and should not be construed to limit the scope of the invention as indicated by the appended claims in any way.

### BRIEF DESCRIPTION OF THE FIGURES

- **Fig. 1:**: Example for a method of producing an expansible hollow part according to the invention. One or several dipping formers (DF) are dipped at least partially into a dipping bath (DB) comprising the elastic biocompatible material in liquefied form. The dipping former may have any shape and can, thus, also be, for example, a medical device.

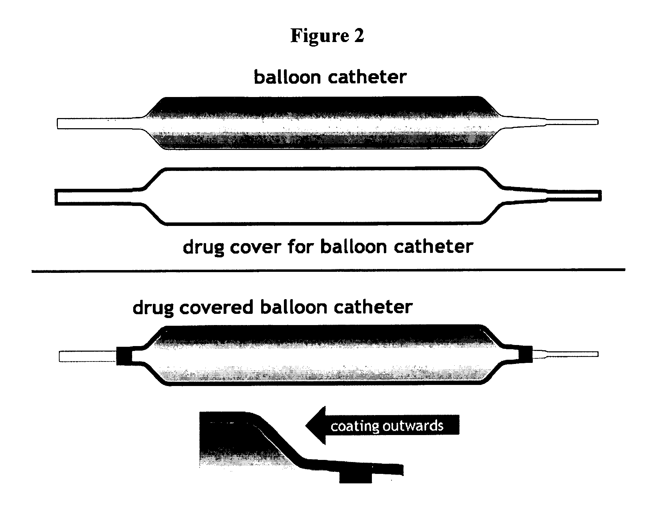

- **Fig. 2:**: Example of an expansible hollow part of the invention, wherein the expansible hollow part is a drug cover which covers a balloon catheter. Such shaped expansible hollow part which is coated with at least one biologically active substance is preferably trimmed after production on both ends and tightly fitted around a medical device such as a balloon catheter. In this figure the biologically active substance is on the outside such that upon expansion of the balloon, the vessel wall (not shown) is contacted with the biologically active substance.

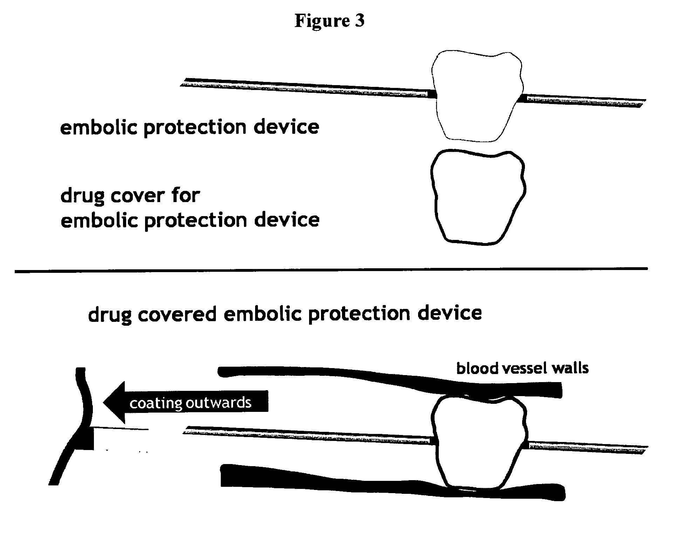

- **Fig. 3:**: Example of an expansible hollow part of the invention, wherein the expansible hollow part is used on an embolic protection device. The at least one biologically active substance is located in the pores and/or in the micro-cavities of the outer surface of the expansible hollow part which covers the embolic protection device. Thus, the biologically active substance is transferred from the expansible hollow part to the vessel cells (i.e., the "landing zone") contacted by the embolic protection device.
- **Fig. 4**: Example of an expansible hollow part of the invention, wherein the expansible hollow part is used for protecting a medical device, for example a hip implant. Preferably, the expansible hollow part comprises at least one biologically active substance on the inner surface of the expansible hollow part. Expansible hollow parts which are coated on the inside can be obtained, for example, by everting the expansible hollow part after coating it with the at least one biologically active substance (for example after step 2 shown in figure 5). When medical devices are stored and/or transported in a protective expansible hollow part as shown, the biologically active substance will be transferred from the expansible hollow part onto the medical device. After shipping and upon use, the expansible hollow part is optionally removed from the medical device which will be covered by the biologically active substance.
- **Fig. 5**: Schematics of a coating process is shown in step 1 and 2. Schematics of mounting the expansible hollow part (in this figure also called "drug cover") onto a medical device is shown in steps 3 and 4. In step 1, the expansible hollow part (in this figure also called "drug cover") is mounted on an expansion tool. Upon expansion, the hollow part is coated, for example on the outside, with at least one biologically active substance (step 2). If the expansible hollow part is to be mounted on a medical device, for example a balloon catheter, the medical device is inserted into the expansion tool as shown (step 3). Next, the expansion tool is retracted which allows the expansible hollow part to shrink onto the medical device (steps 3 and 4).
- **Fig. 6**: Determining surface roughness. The average roughness height of the material surface is measured, e.g. by microscopy.

- **Fig. 7**: The amount of biologically active material which is located in the pores and/or micro-cavities can be determined by e.g. microscopy. A cross section of a region close to the surface of an expansible hollow part of the invention is shown. "A" designates biologically active substance which is located in one exemplified pore/micro-cavity and "B" designates biologically active substance which is located on the surface of the expansible hollow part of the invention but which is not in pores and/or micro-cavities. The biologically active substance can more easily be dissolved from the hollow part when it is in its expanded state (right). This figure is a schematic illustration only and, thus, additional pores and/or micro cavities that may be present in an embodiment of the expansible hollow part of the invention are not shown.

### EXAMPLES

### Example 1:

Experimental data show that short-term application of paclitaxel to human endothelial progenitor cells (EPC) and smooth muscle cells (SCM) leads to apoptosis and therefore inhibition of cell proliferation and migration. This effect is dose and time dependent; rising concentrations in paclitaxel and longer application show higher inhibition of cell proliferation and migration. The expansible hollow part was shown to hold more than 6 µg/mm² paclitaxel in its non-expanded state.

The use of an expansible hollow part which comprised paclitaxel in an amount of between 1 - 6 µg/mm² has proven to be more effective in animal trials.

Thus, a most preferred application of the invention is therefore a paclitaxel-eluting expansible hollow part mounted on a balloon-catheter for the treatment of stenoses or restenoses. A further most preferred application is a metal stent mounted on a paclitaxel-eluting expansible hollow part mounted on a balloon-catheter for the treatment of stenoses or restenoses.

## Claims

1. An expansible hollow part, having at least one opening, which consists of an elastic biocompatible material and which comprises at least one biologically active substance and, optionally, at least one matrix compound, wherein the expansible hollow part is porous and/or comprises micro-cavities in its surface.

2. An expansible hollow part, having at least one opening, which consists of an elastic biocompatible material and which comprises at least one biologically active substance, and, optionally, at least one matrix compound, wherein said expansible hollow part is **characterized in that** it has an inner diameter smaller than 1 cm in its non-expanded state.

3. An expansible hollow part, having at least one opening, which consists of an elastic biocompatible material and which comprises at least one biologically active substance, and, optionally, at least one matrix compound, wherein said expansible hollow part has a wall strength of smaller than 1 mm in its non-expanded state.

4. The expansible hollow part of any of claims 1 to 3, wherein the hollow part is expansible to at least 110% of the circumference of its non-expanded state.

5. The expansible hollow part of any of claims 2 to 4, wherein the expansible hollow part is porous and/or comprises micro-cavities in its surface.

6. The expansible hollow part of any of claims 1 to 5, wherein the maximum pore size of the porous expansible hollow part is 1 µm.

7. The expansible hollow part of any of claims 1 and 3 to 6, wherein the expansible hollow part has an inner diameter smaller than 1 cm in its non-expanded state.

8. The expansible hollow part of any of claims 1 to 7, wherein more than 50% of the biologically active substance is located in the pores and/or in the micro-cavities of the outer surface of the expansible hollow part.

9. The expansible hollow part of any of claims 1 to 7, wherein more than 50% of the biologically active substance is located in the pores and/or in the micro-cavities of the inner surface of the expansible hollow part.

10. The expansible hollow part of any of claims 1 to 7, wherein more than 50% of the biologically active substance is located in the pores and/or in the micro-cavities of the inner and outer surface of the expansible hollow part.

11. The expansible hollow part of any of claims 1 to 7, wherein more than 50% of a first biologically active substance is located in the pores and/or in the micro-cavities of the inner surface of the expansible hollow part and wherein more than 50% of a second biologically active substance is located in the pores and/or in the micro-cavities of the outer surface of the expansible hollow part.

12. The expansible hollow part according to any of claims 1-11, wherein the elastic biocompatible material consists, comprises or essentially consists of a material selected from the group consisting of:
latex, polyvalerolactone, poly-ε-decalactone, polylactic acid, polyglycol acid, polylactide, polyglycolide, co-polymer of polylactide and polyglycolide, poly-ε-caprolactone, polyhydroxy butyric acid, polyhydroxybutyrate, polyhydroxyvalerate, polyhydroxybutyrate-co-valerate, poly(1,4-dioxan-2,3-dione), poly(1,3-dioxan-2-one), poly-para-dioxanone, polyanhydride, polymaleicacidanhydride, polyhydroxymethacrylate, fibrin, polycyanoacrylate, polycaprolactondimethylacrylate, poly-β-maleic acid, polycaprolactonbutylacrylate, multiblockpolymers made of oligocaprolactondiole and oligodioxanondiole, polyetherestermultiblockpolymers made from PEG and polybutylenterephtalate, polypivotolactone, poly-glycolic acid trimethylcarbonate polycaprolactonglycolide, poly(g-ethylglutamate), poly(dth-iminocarbonate), poly(dte-co-dt-carbonat), poly( bisphenol A-iminocarbonate), polyorthoester, poly- glycolic acid -trimethylcarbonate, polytrimethylcarbonate polyiminocarbonate, poly(n-vinyl)-pyrrolidone, polyvinylalcohols, polyesteramide, glycolized polyester, polyphosphoester, polyphosphazene, poly(p-carboxyphenoxy)propane], polyhydroxypentanoic acid, polyethylenoxidpropylenoxid, polyurethane, polyurethane comprising amino acids, polyetherester like polyethyleneoxide, polyalkeneoxalate, polyorthoester, lipids, carrageenane, fibrinogen, starch, collagene, protein-based polymers, polyaminoacids, zein, polyhydroxyalkanoate, pectic acid, actinic acid, carboxymethylsulfate, albumine, hyaluronic acid, chitosane, heparanesulfate, heparine, chondroitinsulfate, dextrane, β-cyclodextrine, copolymers comprising PEG and polypropyleneglycole, gummi arabicum, guar, gelatine, collagen-n-hydroxysuccinimide, phospholipids, polyacrylic acid, polyacrylate, polymethylmethacrylate, polybutylmethacrylate, polyacrylamide, polyacrylonitrile, polyamide, polyetheramide, polyethyleneamine, polyimide, polycarbonate, polycarbourethane, polyvinylketone, polyvinylhalogenide, polyvinylidenhalogenide, polyvinylether, polyisobutylene, aromatic compounds comprising a polyvinyl functional group, polyvinylester, polyvinylpyrollidone, polyoxymethylene, polytetramethyleneoxide, polyethylen, polypropylen, polytetrafluorethylen, polyetherurethane, silicon- polyetherurethane, silicon-polyurethane, silicon-polycarbonat-urethane, polyolefin-elastomers, epdm-rubber, fluorosilicone, carboxymethylchitosane, polyaryletheretherketone, polyetheretherketone, polyethylenterephtalate, polyvalerate, carboxymethylcellulose, cellulose, rayon, rayontriacetate, cellulosenitrate, celluloseacetate, hydroxyethylcellulose, cellulosebutyrate, celluloseacetatebutyrate, ethylvinylacetate, polysulfone, epoxy-resin, abs-resin, silicone like polysiloxane, polydimethylsiloxane, polyvinylhalogens, cellulose-ether, cellulose-triacetate, copolymers mixtures and derivatives thereof.

13. The expansible hollow part according to any of claims 1-12, wherein the biologically active substance is selected from the group consisting of: a vasoconstrictor, a vasodilatator, a muscle relaxant, an antimycotic, a cytotoxic agent, a prodrug of a cytotoxic agent, a virustatic, a physiological or pharmacological inhibitor of mitogens, a cytostatic, a chemotherapeutic, an adrenocorticostatic, a β-adrenolytic, an androgen or antiandrogen, an antianemic, an anabolic, an anaesthetic, an analeptic, an antiallergic, an antiarrhythmic, an antiarterosclerotic, an antibiotic, an antifibrinolytic, an anticonvulsive, an angiogenesis inhibitor, an anticholinergic, an enzyme, a coenzyme, an antihistaminic, an antihypertensive, an antihypotensive, an anticoagulant, an antiseptic, an antihemorrhagic, a beta-receptor antagonist, a calcium channel antagonist, an antimyasthenic, an antiphlogistic, an antipyretic, a glucocorticoid, a haemostatic, an immunoglobuline or its fragment, a chemokine, a mitogen, a cell differentiation factor, a hormone, an immunosuppressant, an immunostimulant, a mineralcorticoid, a narcotic, a vector, a peptide, a (para)-sympathicomimetic, a (para)-sympatholytic, a protein, a cell, a sedating agent, a spasmolytic, a wound-healing substance, and combinations thereof.

14. The expansible hollow part according to any of claims 1-13, wherein the expansible hollow part comprises at least 0.5 µg of the biologically active substance per square millimetre surface in its non-expanded state.

15. The expansible hollow part according to any of claims 1-14, wherein the matrix compound is selected from a group consisting of polyvalerolactone, poly-ε-decalactone, polylactic acid, polyglycolic acid, polylactide, polyglycolide, copolymers of polylactide and polyglycolide, poly- ε -caprolactone, polyhydroxylbutyric acid, polyhydroxybutyrate, polyhydroxyvalerate, polyhydroxybutyrate-co-valerate, poly(1,4-dioxane-2,3-dione), poly(1,3-dioxane-2-one), poly-para-dioxanone, polyanhydride, polymaleicacidanhydride, polyhydroxymethacrylate, fibrin, polycyanoacrylate, polycaprolactondimethylacrylate, poly-b-maleic acid polycaprolactonbutylacrylate, multi-block polymers of oligocaprolactondiole and oligodioxanonediole, polyetherestermultiblockpolymers made of peg und polybutylene terephthalate, polypivotolactone, polyglycolic acid trimethylcarbonate polycaprolactonglycolide, poly(g-ethylglutamate), poly(dth-lminocarbonate), poly(dte-co-dt-carbonate), poly( bisphenol a-iminocarbonate), polyorthoester, polyglycolic acid trimethylcarbonate, polytrimethylcarbonate, polyiminocarbonate, poly(n-vinyl)-pyrrolidone, polyvinylalcohol, polyesteramide, glycolated polyester, polyphosphoester, polyphosphazene, poly[p-carboxyphenoxy)propane], polyhydroxypentanoic acid, polyethyleneoxidepropyleneoxide, polyurethane, polyurethane having amino acids in the backbone, polyetherester like polyethyleneoxide, polyalkeneoxalate, lipid, carrageenane, fibrinogen, starch, collagene, polymers comprising protein, protein, polyaminoacid, synthetic polyaminoacid, zein, polyhydroxyalkaneoate, pectic acid, actinic acid, carboxymethylsulfate, albumin, hyaluronic acid, chitosan und derivatives thereof, heparanesulfate und ist derivatives, heparine, chondroitinsulfate, dextrane, β-cyclodextrine, copolymers of peg and polypropyleneglycol, gummi arabicum, guar, gelatine, collagen-n-hydroxysuccinimide, phospholipid, polyacrylic acid, polyacrylate, polymethyl methacrylate, polybutyl methacrylate, polyacrylamide, polyacrylonitrile, polyamide, polyetheramide, polyethyleneamine, polyimide, polycarbonate, polycarbourethane, polyvinylketone, polyvinylhalogenide, polyvinylidenhalogenide, polyvinylether, polyisobutylene, polyvinyl compounds, polyvinyl ester, polyvinylpyrollidone, polyoxymethylene, polytetramethylene oxid, polyethylene, polypropylene, polytetrafluoroethylene, polyetherurethane, silicone-polyetherurethane, silicone-polyurethane, silicone-polycarbonate-urethane, polyolefin, epdm-rubber, fluorosilicone, carboxymethylchitosane, polyaryletheretherketone, polyetheretherketone, polyethylene terephthalat, polyvalerate, carboxymethylcellulose, rayon, rayontriacetate, cellulose nitrate, cellulose acetate, hydroxyethylcellulose, cellulosebutyrate, celluloseacetatbutyrate, ethylvinylacetate, polysulfone, epoxy resin, abs-resin, silicone like polysiloxane, polydimethylsiloxane, polyvinylhalogene, cellulose ether, cellulose triacetate, chiosane, combinations and copolymers of combinations thereof.

16. Method of producing an expansible hollow part having at least one opening, which consists of an elastic biocompatible material and which comprises at least one biologically active substance and, optionally, at least one matrix compound, comprising the steps:
(a) expanding the expansible hollow part to at least 110% of its non expanded circumference, and,
(b) contacting the outer surface of the expansible hollow part with at least one biologically active substance and/or at least one matrix compound.

17. The method according to claim 16 further comprising at least one additional step selected from the steps consisting of:
(c) relaxing the expansible hollow part so that its circumference is smaller than the circumference of the expansible hollow part in step (a);
(d) before carrying out step (a), forming the expansible hollow part in a dipping bath comprising the elastic biocompatible material in liquefied form and optionally, a biologically active substance; and
(f) mechanically everting the expansible hollow part.

18. The method according to claim 17, wherein the steps (a) and (b) or (b) and (c) are carried out simultaneously.

19. The method according to claim 17 or 18, wherein
(v) step (e) is carried out before step (a) and/or after step (b) and/or after step (c);
(vi) steps (d), (a) and (b) are carried out in that order;
(vii) steps (d), (e), (a) and (b), are carried out in that order; or
(viii) steps (b), (e), (b) are carried out in that order, whereby in the first step (b) a different biologically active substance is used than the second step (b).

20. The method according to any of claims 16-19, wherein the expansion in step (a) is carried out using an expansible medical device or a mechanical tool.

21. An expansible hollow part producible by the method of any of claims 16-20.

22. A medical device covered at least partially by the expansible hollow part according to any of claims 1-15 or 21.

23. Medical device according to claim 22, wherein the medical device is selected form the group consisting of a stent, a balloon catheter, a probe, a prosthesis, an endoscope, a pace maker, a heart defibrillator and a perfusion catheter.

24. Medical device according to claim 23, wherein the medical device is a balloon catheter; and wherein the expansible hollow part is covered at least partially by a stent.

25. Medical device according to claim 23, wherein the medical device is a stent; and wherein the stent is on a balloon catheter.

26. Medical device according to any of claims 23-25, wherein the balloon catheter comprises a hot balloon, a cold balloon, an occlusion balloon, a valvuloplasty-balloon and/or a protection device.

27. Medical device according to any of claims 23-25, wherein the balloon catheter is a perfusion catheter which comprises a hot balloon or a cold balloon.

28. Medical device according to any of claims 23-27, wherein the balloon catheter and the expansible hollow part are connected to each other by at least one clamping piece.

29. Medical device according to any of claims 22-28, wherein the circumference of the medical device in its non-expanded state is greater than the inner circumference of the expansible hollow part in its non-expanded state.

30. A kit-of-parts comprising at least one expansible hollow part according to any of claims 1-15 or 21 and at least one medical device.

31. Kit-of-parts according to claim 30, wherein the medical device is selected from the group consisting of a stent, a balloon catheter, a probe, a prosthesis, an endoscope, a pace maker, a heart defibrillator and a perfusion catheter.

32. Kit-of-parts according to any of claims 30 or 31, wherein the circumference of the medical device in its non-expanded state is greater than the inner circumference of the expansible hollow part in its non-expanded state.

33. Use of an expansible hollow part according to any of claims 1-15 or 21 for the preparation of an enhanced balloon catheter for the treatment of a disease or a medical insufficiency selected from the group consisting of a stenosis, a restenosis, a stricture, a defective bypass craft, a thrombosis, a dissection, a tumor, a calcification, an arteriosclerosis, an inflammation, an autoimmune response, a necrosis, an injured anastomosis, a lesion, an allergy, a wart, a hyperproliferation, an infection, a scald, an edema, a coagulation, a cicatrization, a burn, a frostbite and a lymphangitis.

34. Expansible hollow part according to any of claims 1-15 or 21 for the use as a therapeutical device for the treatment of a disease or a medical insufficiency selected from the group consisting of stenosis, restenosis, a stricture, a defective bypass craft, a thrombosis, a dissection, a tumor, a calcification, an arteriosclerosis, an inflammation, an autoimmune response, a necrosis, an injured anastomosis, a lesion, an allergy, a wart, a hyperproliferation, an infection, a scald, an edema, a coagulation, a cicatrization, a burn, a frostbite and a lymphangitis.

35. Use of an expansible hollow part according to any of claims 1-15 or 21 for protecting a medical device or a biological tissue from mechanical stress, thermal stress, chemical stress and/or micro organisms.
